# EUROPEAN PATENT APPLICATION

(11) **EP 2 987 482 A1**
(43) Date of publication of application: **24.02.2016**
(21) Application number: 14002916.6
(22) Date of filing: 22.08.2014
(51) Int. Cl.: A61K 9/00, A61K 9/20, A61K 31/4196

(54) **Soluble and dispersible pharamaceutical deferasirox formulation**

(71) Applicant: Santa Farma Ilaç Sanayi A.S., 34382 Istanbul (TR)
(72) Inventor: Aksoy, Ediz, Istanbul (TR); Öztuna, Banu, 34382 Istanbul (TR); Kanik, Bayram, Istanbul (TR); Ünver, Levent, Istanbul (TR); Yildirim, Güher Isik, Istanbul (TR); Öner, Levent, 06610 Ankara (TR)
(74) Representative: Bulut, Pinar

(57) **Abstract**

The present invention relates to a soluble and dispersible pharmaceutical composition for oral administration comprising deferasirox. More specifically, the present invention relates to a soluble and dispersible pharmaceutical composition for oral administration comprising deferasirox and a weak alkali and/or alkali agent in an acceptable carrier such that the said composition has a pH of 7.4-10.0 when dispersed in water at 15-25°C, and solubility of deferasirox greater thin 0.04 mg/ml.

## Description

### FIELD OF INVENTION

The present invention relates to a soluble and dispersible pharmaceutical composition for oral administration comprising deferasirox. More specifically, the present invention relates to a soluble and dispersible pharmaceutical composition for oral administration comprising deferasirox and a weak alkali and/or alkali agent in an acceptable carrier such that the said composition has a pH of 7.4-10.0 when dispersed in water at 15-25°C, and solubility of deferasirox greater than 0.04 mg/ml.

### BACKGROUND OF THE INVENTION

Deferasirox (4-[3,5-bis(2-hydroxyphenyl)-[1,2,4]triazol-1-yl]benzoic acid) as shown in Formula I in its free acid form, the salts thereof and its crystalline forms are disclosed in WO 97/49395.

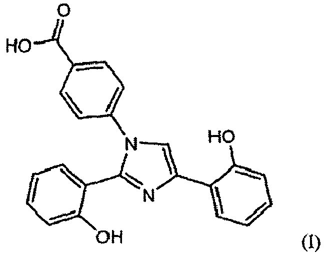

Deferasirox, currently marketed under the trade name Exjade® (Novartis), is an orally active iron chelator that is indicated in the treatment of iron overload in transfusion dependent anemias, in particular thalassemia major, thalassemia intermediate and in sickle cell disease to reduce iron-related morbidity and mortality, in patients having an age of two years and older.

Deferasirox is highly water-insoluble and highly lipid-soluble, and is observed to possess good permeability. Hence, according to the current Biopharmaceutical Classification System (BCS), deferasirox has been classified as a Class II drug. It exhibits a high pH-dependent solubility, meaning it remains practically insoluble until the buffer strength is altered to get optimal dissolution profile.

Yalkowsky SH and Dannenfelser RM (1992) reports a solubility of 0.038 mg/ml for deferasirox in water at 37°C, while the summary of product characteristics (SmPC) of Exjade® dispersible tablets reports a solubility about 40 mg/L at the physiological pH of the intestine.

Deferasirox is commercially available as dispersible tablet (Exjade®) for oral administration containing 125 mg, 250 mg and 500 mg of deferasirox per tablet. Prior to administration the tablet is dispersed by stirring in an aqueous medium (i.e., a glass of water or orange or apple juice, 100-200 ml), and the resulting fine suspension then is administered by the patient via oral route. By this means, high dosage strengths are administered easily without being swallowed in undivided form or without being chewed, in particular by children and elderly.

The recommended initial daily dose of deferasirox is 20 mg/kg body weight, and the dose is adjusted up to a maximum of 30 mg/kg body weight. Doses are calculated and rounded to the nearest whole tablet size, and in order to have a clinical benefit, the daily dosage of deferasirox is recommended to be maintained on the higher side.

Typically, prescribed daily dosages of deferasirox for the treatment of thalassemia are high, e.g. 5-40 mg/kg of body weight/day in adults. In children, the dosage is preferably 5-30 mg/kg of body wieght/day.

The prior art includes various number of patents/patent applications relating to the deferasirox dispersible tablets.

WO 2004/035026 (Novartis AG) discloses a dispersible tablet comprising deferasirox in an amount of 5%-40% and a disintegrant in an amount of 10-35% based on the total weight of the tablet. More specifically, this application relates to dispersible tablets containing deferasirox in an amount 100-600 mg/tablet.

WO 2005/097062 (Novartis AG) relates to a dispersible tablet comprising deferasirox in an amount of 42-65% based on the total weight of the tablet. More specifically, this application is relates to dispersible tablets containing higher amount deferasirox (i.e., 900-1100 mg/tablet).

WO 2007/045445 (Novartis AG) relates to a dispersible tablet comprising deferasirox in an amount of 42-65%, at least one filler in a total amount of about 35-45%, at least one disintegrant in a total amount of about 2-8%, at least one binder in a total amount of about 1-5%, at least one surfactant in a total amount of about 0.01-1%, at least one glidant in a total amount of about 0.1-5%, and at least one lubricant in a total amount of about 0.45-0.85% by total weight of the tablet. More specifically, this application relates to dispersible tablets containing 900-1100 mg of deferasirox per tablet.

WO 2009/067557 (Teva) discloses a process of preparing deferasirox formulations having sufficiently high dissolution rate and good bioavailability wherein said process comprises co-milling deferasirox with at least two pharmaceutically acceptable excipients, wherein one excipient is a surfactant, in the absence of any solvent.

WO 2010/035282 (Matrix Lab) discloses oral pharmaceutical composition comprising deferasirox in the form of a dispersible tablet wherein the active ingredient has a mean particle size less than about 100 µm and is present in an amount greater than 66% by weight based on total weight of the tablet.

WO 02/36101 (Laboratorios Belmac) relates to a pharmaceutical formulation of paracetamol with minimum effervescent effects that is dispersible and soluble in cold water, consisting a mixture of paracetamol and citric acid in combination with a weak alkali agent to adjust a pH of 5.0-5.5 when dispersed in water.

### DESCRIPTION OF THE INVENTION

Based on the prior art, the object of the invention is to develop not only dispersible but also soluble pharmaceutical composition comprising deferasirox that would exhibit acceptable dissolution and absorption. More specifically, the main object of the invention is to develop a soluble and dispersible pharmaceutical composition for oral administration comprising deferasirox and a weak alkali and/or alkali agent in an acceptable carrier such that the said composition has a pH of 7.4-10.0 when dispersed in water at 15-25°C, and solubility of deferasirox greater than 0.04 mg/ml.

By means of the detailed description below, a pharmaceutical formulation to be used via oral route after dispersion is developed in order to reach all the objects of the invention.

In a preferred embodiment of the invention, the said invention is achived by combining deferasirox with a weak alkali and/or alkali agent and at least one excipient. Examples of weak alkali and/or alkali agents are, but not limited to: sodium carbonate, sodium bicarbonate, calcium carbonate, calcium bicarbonate, sodium hydroxide, potassium hydroxide, magnesium oxide.

In a preferred embodiment of the invention, the weak alkali and/or alkali agent is sodium carbonate. The pharmaceutical composition comprises sodium carbonate in an amount between 4% and 16% by weight based on the total weight of the composition. Preferably the sodium carbonate present in an amount of from 6% to 12% by weight based on the total weight of the composition. More preferably the amount of sodium carbonate is approximately 8% by weight based on the total weight of the composition.

In a preferred embodiment of the invention, the said pharmaceutical excipients are diluent, binder, suspending agent, disintegrant, lubricant, surface active agent, glidant, aromatic agent, sweetening agent, preservative, coloring agent.

The diluent may be present individually or in combination in the composition. Examples of diluents are, but not limited to: lactose anhydrous, lactose monohydrate, lactose spray-dried, ethyl cellulose, microcrystalline cellulose, mannitol, sorbitol.

The binder may be present individually or in combination in the composition. Examples of binders are, but not limited to: polyvinylpyrrolidone, cross-linked polyvinylpyrrolidone, hydroxypropyl cellulose, hydroxypropylethyl cellulose, hydroxypropylmethyl cellulose, microcrystalline cellulose.

The suspending agent may be present individually or in combination in the composition. Examples of suspending agents are, but not limited to: microcrystalline cellulose, hydroxypropyl cellulose, carboxymethyl cellulose, silicon dioxide, gum arabic, xanthan gum, polisorbates, glyceryl monostearate.

The disintegrant may be present individually or in combination in the composition. Examples of disintegrants are, but not limited to: polyvinylpyrrolidone, cross-linked polyvinylpyrrolidone, carmellose sodium, carmellose potassium, croscarmellose sodium, croscarmellose potassium, microcrystalline cellulose.

The lubricant may be present individually or in combination in the composition. Examples of lubricants are, but not limited to: magnesium stearate, aluminum stearate, calcium stearate, polyethylene glycol, talc, glyceryl monostearate, glyceryl dibehenate, glyceryl palmitostearic ester, polyoxyethylene glycol, sodium stearyl fumarate.

The surface active agent may be present individually or in combination in the composition. Examples of surface active agents are, but not limited to: sodium lauryl sulfate, betain, quarternary amonium salts, polisorbates, sorbitan esters, poloxamer.

The glidant may be present individually or in combination in the composition. Examples of glidants are, but not limited to: silicon dioxide, colloidal silicon dioxide, magnesium trisilicate, cellulose, starch, talc.

The aromatic agent may be present individually or in combination in the composition. Examples of aromatic agents are, but not limited to: vanilla, orange, lemon, mandarin, grapefruit, tutti frutti, banana, strawberry, cherry, raspberry, green apple, peach like fruit extracts.

The sweetening agent may be present individually or in combination in the composition. Examples of sweetening agents are, but not limited to: sugars like aspartame, acesulfame potassium, sodium cyclamate, maltodextrine, dextrose, taumatine, sucralose, saccharin, sucrose, glucose, fructose, saccharose and sugar alcohols like mannitol, sorbitol, xylitol; and the like.

The preservative may be present individually or in combination in the composition. Examples of preservatives are, but not limited to: parabens, sodium benzoate, citric acid.

The coloring agent may be present individually or in combination in the composition. Examples of coloring agents are, but not limited to: pigments, dyes, titanium dioxide, iron oxides.

In a preferred embodiment of the invention, the said pharmaceutical formulation is formulated in a single dosage form for oral administration such as tablet, powder, granules or a combination thereof.

### DETAILED DESCRIPTION OF THE INVENTION

In the scope of the present invention, pharmaceutical compositions comprising deferasirox combined with sodium carbonate in a suitable carrier were prepared.

Table 1 below, without being limited to the compounds and their amounts mentioned, illustrates the pH values and solubility data of a pharmaceutical composition comprising 500 mg of deferasirox combined with various amounts of sodium carbonate in a suitable carrier when the said composition was dissolved using a magnetic stirrer for 3 minutes.

**TABLE 1**

| **Amount of sodium carbonate (mg)** | **pH of media** | **Solubility of deferasirox in water (mg/ml)** |
|---|---|---|
| 40 | 8.13 | 0.27 |
| 60 | 8.33 | 0.46 |
| 80 | 8.44 | 0.65 |
| 100 | 8.65 | 0.76 |
| 120 | 8.77 | 0.9 |
| 140 | 8.84 | 1.03 |
| 160 | 8.91 | 1.09 |

For example, according to the present invention a pharmaceutical composition comprising 500 mg of deferasirox combined with 80 mg of sodium carbonate has reached a solubility of 0.65 mg deferasirox per ml of water (i.e., >0.04 mg/ml solubility of deferasirox alone) and the pH of the medium was measured to be 8.44.

### Example 1:

Pharmaceutical composition containing 125 mg, 250 mg, 500 mg, or 1000 mg of deferasirox given below was prepared by the following procedure.

Deferasirox and Sodium laurylsulfate were sifted and uniformly mixed. The blend was wet granulated by povidone solution and the granules were dried.

The dried granulation was passed through a suitable sieve.The granules were blended with previously milled Crospovidone XL, Microcrystalline cellulose, Lactose spray- dried, Sodium carbonate and Colloidal silicon dioxide.The blended powder was lubricated with Magnesium stearate.

### Example 1:

| **Process step** | **Component** | **Quantity per dosage unit (mg)** | | | | **% (w/w)** |
|---|---|---|---|---|---|---|
| **Inner Phase** | Deferasirox | 1000.0 | 500.0 | 250.0 | 125.0 | 50.0 |
| | Polyvinylpyrrolidon K30 | 60.0 | 30.0 | 15.0 | 7.5 | 3.0 |
| | Sodium laurylsulfate | 2.0 | 1.0 | 0.5 | 0.25 | 0.1 |
| **Outer Phase** | Crospovidone XL | 180.0 | 90.0 | 45.0 | 22.5 | 9.0 |
| | Microcrystalline cellulose | 200.0 | 100.0 | 50.0 | 25.0 | 10.0 |
| | Lactose spray- dried | 378.0 | 189.0 | 94.5 | 47.25 | 18.9 |
| | Sodium carbonate | 160.0 | 80.0 | 40.0 | 20.0 | 8.0 |
| | Colloidal silicon dioxide | 10.0 | 5.0 | 2.5 | 1.25 | 0.5 |
| | Magnesium stearate | 10.0 | 5.0 | 2.5 | 1.25 | 0.5 |
| **Total** | | **2000.0** | **1000.0** | **500.0** | **250.0** | **100.0** |

Example 1 for pharmaceutical composition containing 125 mg, 250 mg, 500 mg, or 1000 mg of deferasirox, illustrates the invention without being limited to the compounds and their amounts mentioned.

## Claims

1. A soluble and dispersible oral pharmaceutical composition comprising deferasirox and a weak alkali and/or alkali agent in an acceptable carrier **characterized in that** said composition has a pH of 7.4-10.0 when dispersed in water at 15-25°C, and solubility of deferasirox greater than 0.04 mg/ml.

2. The pharmaceutical composition according to claim 1 wherein the weak alkali and/or alkali agent is preferably selected from sodium carbonate, sodium bicarbonate, calcium carbonate, calcium bicarbonate, sodium hydroxide, potassium hydroxide, magnesium oxide or admixtures thereof.

3. The pharmaceutical composition according to claim 1 wherein the weak alkali and/or alkali agent is sodium carbonate.

4. The pharmaceutical composition according to claim 1 wherein sodium carbonate is present in an amount between 4% and 16% by weight based on the total weight of the composition.

5. The pharmaceutical composition according to claim 1 wherein sodium carbonate is present in an amount between 6% and 12% by weight based on the total weight of the composition.

6. The pharmaceutical composition according to claim 1 wherein sodium carbonate is present in an amount approximately 8% by weight based on the total weight of the composition.

7. The pharmaceutical composition according to claim 1 wherein the composition may comprise excipients such as disintegrants, lubricants, surface active agents, or admixtures thereof.

8. The pharmaceutical composition according to claim 7 wherein the disintegrant is preferably selected from povidone, crospovidone, cross polyvinyl pirolidon, carmellose sodium, carmellose potassium, croscarmellose sodium, croscarmellose potassium, microcrystalline cellulose or admixtures thereof.

9. The pharmaceutical composition according to claim 7 wherein the lubricant is preferably selected from magnesium sterate, aluminium stearate, calcium stearate, polyethylene glycol, talc, glyceryl monostearate, glyceryl dibehenate, glyceryl palmitostearic ester, polyoxyethylene glycol, sodium stearyl fumarate or admixtures thereof.

10. The pharmaceutical composition according to claim 7 wherein the surface active agent is preferably selected from the group consisting of sodium lauryl sulfate, betain, quarterner ammonium salts, polysorbates, sorbitan esters and poloxamer or admixtures thereof.

11. The pharmaceutical composition according to claim 7 wherein the composition may further comprise binders, glidants, anti-adherents, colorants, taste correctors, sweeeteners, flavoring agents or mixtures thereof.

12. The pharmaceutical composition according to any one of the preceding claims contains deferasirox in an amount of 125 mg -1500 mg per oral dosage unit to meet the recommended initial daily dose of deferasirox of 20 mg/kg body weight.

13. The pharmaceutical composition according to any one of the preceding claims is formed into a pharmaceutical dosage form suitable for oral administration such as tablets, granules, powder or admixtures thereof.
